# EUROPEAN PATENT APPLICATION

(11) **EP 2 997 963 A1**
(43) Date of publication of application: **23.03.2016**
(21) Application number: 14782293.6
(22) Date of filing: 10.04.2014
(51) Int. Cl.: A61K 8/96, A23L 33/10, A61K 36/03

(54) **ANTIOXIDANT EXTRACT FROM BROWN MACROALGAE AND METHOD FOR OBTAINING SAME**

(30) Priority: 12.04.2013 ES 201330523
(71) Applicant: Universidade de Santiago de Compostela, 15782 Santiago de Compostela (ES)
(72) Inventor: SINEIRO TORRES, Jorge, E-15782 Santiago de Compostela (La Coruña) (ES); SÁNCHEZ GUERRERO, Marivel, E-15782 Santiago de Compostela (La Coruña) (ES); NÚÑEZ GARCÍA, María José, E-15782 Santiago de Compostela (La Coruña) (ES)
(74) Representative: ABG Patentes, S.L.
(86) International application number: PCT/ES2014/070288
(87) International publication number: WO 2014/167162

(57) **Abstract**

The invention relates to a method for obtaining antioxidant extracts from macroalgae using ultrasound-assisted continuous aqueous extraction. The process can be performed using fresh alga or dry alga, after resuspending same in water. A suspension of alga in water is prepared with a solid concentration of between 10 and 30%. The mixture is fed to an ultrasonic disruption system. The extract is filtered and lyophilized, obtaining total polyphenol concentrations of 62.4 mg eq. of phloroglucinol/g of lyophilisate with *Bifurcaria bifurcata* and 44 mg eq. of phloroglucinol/g of lyophilisate with *Ascophyllum nodosum.* The extract can be used as an ingredient in cosmetic and food formulations.

## Description

### TECHNICAL FIELD OF THE INVENTION

The present invention relates to the extraction of compounds from macroalgae. More specifically, the invention relate to the extraction of antioxidants from brown macroalgae.

### STATE OF THE ART

Antioxidants are secondary metabolites present in macroalgae, plants, and particularly fruits. They are compounds that inhibit or prevent oxidation of a substrate. In the food, pharmaceutical and cosmetics industry are used primarily synthetic antioxidant compounds, such as butylated hydroxytoluene (BHT), propyl gallate or butyrohydroxyanisole (BHA). Natural antioxidants like those originating from grape extract, rosemary extract, cocoa extract, etc., are being accepted better due to their low toxicity and high antioxidant activity. A large family of antioxidant compounds are phlorotannins present in macroalgae which show good antioxidant activity.

Several protocols for the extraction of phlorotannins have been proposed, among which it is possible to mention sequential liquid-liquid partitions, using primarily organic solvents such as methanol, hexane, dichloromethane, chloroform, ethyl acetate and butanol, in addition to purifications in chromatographic columns, such as Antioxidant Effects of Phlorotannins Isolated from Ishige okamurae in Free Radical Mediated Oxidative Systems, Yanping, Z., Zhong-Ji Q., Yong L., Moon-Moo K., Sang-Hoon, L. & Se-Kwon K, J. Agric. Food Chem., 56, 7001-7009, 2008; Phenolic Compounds in the Brown Seaweed Ascophyllum nodosum: Distribution and Radical-scavenging Activities, Audibert, L., Fauchon, M., Blanc, N., Hauchard, D. & Ar Galla, E, Phytochemical analysis, 21, 399-405, 2010; Chemical components and its antioxidant properties in vitro: an edible marine brown alga Ecklonia cava, Li, Y., Qian, Z.J., Ryu, B., Lee, S.H., Kim, M.M. and Kim, S.K., Bioorg Med Chem 17, 1963-1973, 2009; Distribution and radical scavenging activity of phenols in Ascophyllum nodosum (Phaeophyceae), Breton, F., Cérantola, S. & Ar Gall E., Journal of Experimental Marine Biology and Ecology, 399, 167-172, 2011. However these processes generate residues that are not allowed in food and cosmetics, such as methanol or organohalogen compounds. Furthermore, if a polarity that is lower than that of water is required, the hydroalcoholic mixtures are an option as opposed to petroleum solvents.

The use of 70% acetone and ethyl acetate as solvents was reported in Phlorotannins as Radical Scavengers from the Extract of Sargassum ringgoldianum, Nakai, M., Kageyama, N., Nakahara, K. & Miki, W., Marine Biotechnology, 8, 409-414, 2006). A first lipid material removal phase with hexane and subsequent extraction of phlorotannins with acetone-water at 70% was used in High-performance liquid chromatographic analysis of phlorotannins from the brown alga Fucus vesiculosus, Koivikko, R., Loponen, J., Pihlaja, K. and Jormalainen, V., Phytochem. Anal. 18, pp. 326-332, 2007. Pure ethanol and 60% ethanol have been used for extraction of phlorotannins in species such as *Ascophyllum nodosum* and Fucus vesiculosus, followed by liquid-liquid partition with petroleum ether or dichloromethane (Evaluation of quantitative methods for the determination of polyphenols in algal extracts, Parys, R., Rosenbaum, A., Kehraus, S., Reher, G., Glombitza, K-W and König, G.M. J. Nat. Prod., 1865-1870, 2007.

### DESCRIPTION OF THE INVENTION

The extracts obtained from processing brown macroalgae, such as *Bifurcaria bifurcata* and *Ascophyllum nodosum*, can be employed for food, cosmetic and/or pharmaceutical use due to their fucoidan and phlorotannin content. The high fucoidan content leads to these extracts having a wetting capability on skin, whereas the antioxidant power is due to the presence of phlorotannins.

Obtaining a natural product that does not have a strong characteristic aroma is desirable for use as a cosmetic ingredient in order to not interfere with other desired uses, which is favored by aqueous extraction and by not using organic solvents. Another desirable feature of the process of extraction of extracts is for the solvents that are used to be inexpensive, renewable, having little or no toxicity and for the handling thereof to not be dangerous. Crude extracts or isolated fractions often show greater antioxidant activity than synthetic antioxidants such as BHA (butyrohydroxyanisole) or BHT (butyrohydroxytoluene).

The method object of the present invention allows using water to obtain a stable product that is easy to handle and add to different products and free of trace solvents.

The method of extraction of the antioxidant from a brown macroalga comprises:
a) If fresh macroalga is used, it is subjected to washing with running water, removing sand and epiphytes, and is then dried with absorbent paper. If dry alga is used, this process of cleaning with water is not necessary.
b) The dry or fresh alga is then mixed with water at a liquid/solid (L/S) ratio of between 3 and 5 g/g.
c) The mixture from the preceding step is then subjected to a grinding process, preferably in a blade grinder, until reducing the particle size between 0.5 and 2 mm for dry macroalga and between 0.5 and 3 mm for fresh macroalga.
d) Mixing the ground macroalga with a liquid solvent comprising pure ethanol or ethanol:water mixtures at a 1:1 v/v ratio, at a liquid/solid (L/S) ratio of between 5 and 15 (g/g) when starting from fresh macroalga and between 50 and 150 (g/g) when starting from dry macroalga,
e) subjecting the mixture to a cell disruption process by continuously applying ultrasound.
f) The solid algal residue obtained in the preceding step is separated by sedimentation and subsequent centrifugation or filtration.
g) The ethanol or 50% of the water, only if water had been used, is removed under vacuum without exceeding the temperature of 40°C.
h) The concentrated extract is lyophilized to obtain a solid extract.

The product is stored cooled to less than 5°C and protected from the light to prevent it from being altered.

In another aspect, the invention relates to a stable antioxidant extract obtained from processing brown macroalgae, more specifically macroalgae algae of the *Bifurcaria bifurcata, Ascophyllum nodosum, Saccorhiza polyschides* and *Sargassum muticum* species.

### BREVE DESCRIPTION OF THE DRAWINGS

The embodiments shown in detail in the drawings are illustrated by way of non-limiting example:
Figure 1 shows the diagram for ultrasound-assisted cell disruption in a continuous macroalgal feed system.
Figure 2 shows the detection of phlorotannins by means of C18 reversed-phase column HPLC.
Figure 3 shows the ultraviolet profile of the *Bifurcaria bifurcata* extract, with a characteristic phlorotannin profile that is similar to that of phloroglucinol.

### EMBODIMENT OF THE INVENTION

In a particular embodiment the invention relates to an aqueous antioxidant extract obtained from brown macroalgae characterized by a) a carbohydrate content between 33 and 156 mg of glucose/g of lyophilizate; b) a fucoidan content, said fucoidans expressed as total sulfates after digesting the sample, between 44 and 118 mg of sulfates/g of lyophilizate; c) a phlorotannin content, said phlorotannins expressed as phloroglucinol, between 12 and 62.4 mg equivalents of phloroglucinol/g of lyophilizate; and d) an alginate content between 10 and 55 mg equivalents of glucuronic acid/g of lyophilizate.

In another aspect, the invention relates to a cosmetic and/or food composition comprising the antioxidant extract object of the present invention.

An example of the application of the method of extraction of antioxidants from *Bifucaria Bifurcata* is mentioned by way of example and without limiting the scope of protection. The extraction is performed continuously and in temperature conditions less than 40°C to prevent a reduction in the antioxidant power or the amount of the compounds that are extracted.

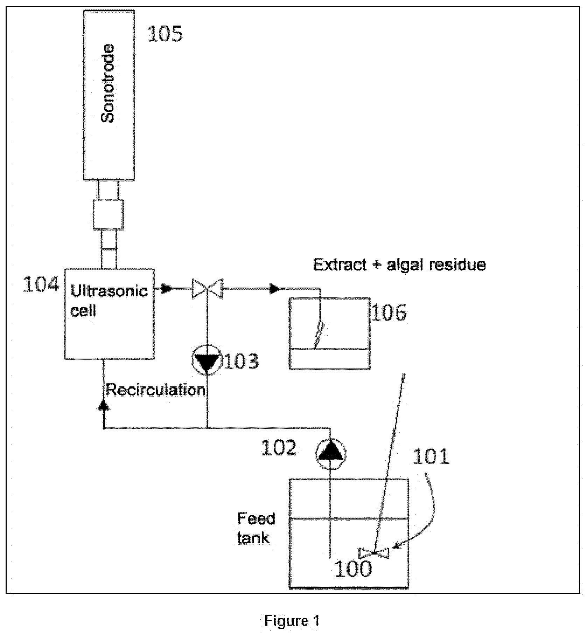

Figure 1 shows the diagram for ultrasound-assisted cell disruption in a continuous macroalgal feed system made up of an ultrasound generating unit including a sonotrode (105) arranged in a flow-through cell (104), which is fed by means of a main peristaltic pump (102) and another recirculating pump (103).

In a particular embodiment of the invention the process of extraction of the aqueous antioxidant compound comprises the following steps:
a) 120 g of fresh macroalga were washed with water fit for drinking and subsequently with distilled water.
b) 300 ml of water were added.
c) The macroalga was pre-ground by operating a blade mixer for 10 min, obtaining a particle size of less than 3 mm.
d) Water was added to the preparation obtained in the preceding step until obtaining an L/S ratio of 10, and it was introduced in a feed tank (100) which is mixed by means of an agitator (101). One of the extraction conditions described in Table 1, which summarizes a 2² factorial experimental design with 4 center points, was selected.
e) The continuous ultrasonic extraction conditions considered the parameters of power (as amplitude or percentage of nominal power applied) and ratios between the recirculation of the inflow rate of the algal broth into the flow-through cell (sonication or ultrasonic cell) (104) in which a sonotrode (105) continuously applies ultrasound to the mixture with a power density in the range of between 3 and 13 W/cm³. Table 1 summarizes the combination of treatments performed for the experimental design. The extraction set-up requires an ultrasonic-assisted extraction system such as the one shown in Figure 1.
f) The sedimentable solid residue was left to settle and was separated from the supernatant by decanting.
g) The next step consisted of centrifuging and separating the supernatant again. The supernatant has a volume of 1200 ml, which was reduced to 500 ml by vacuum evaporation at 30°C or at room temperature,
h) A lyophilized extract (106) was obtained which, in this particular embodiment, comprises a total volume of 1400 ml in which and finally to obtain a solid and dry extract.

**Table 1.- 4 center point, 2² experimental design. Amplitude adjustment codes -1, 0 and 1 correspond to amplitudes of 50%, 70% and 90%. Recirculation ratio codes -1, 0 and +1 correspond to 1, 1.5 and 2, the recirculation flow rate being constant of 300 ml/min**

| Amplitude | Recirculation ratio | Power W/cm³ | Residence time in cell(s) |
|---|---|---|---|
| 1 | 1 | 13 | 9.2 |
| 1 | -1 | 13 | 6.9 |
| -1 | 1 | 7.2 | 9.2 |
| -1 | -1 | 7.2 | 6.9 |
| 0 | 0 | 10.1 | 8.28 |
| 0 | 0 | 10.1 | 8.28 |
| 0 | 0 | 10.1 | 8.28 |
| 0 | 0 | 10.1 | 8.28 |

The yield of extraction on a wet basis from fresh macroalga is within a range of 2.9 - 6.6% (w/w); whereas the yield of extraction for dry alga is within the range between 27.7 and 57.3% (w/w); the yields for each of the algae species analyzed are included in Table 2.

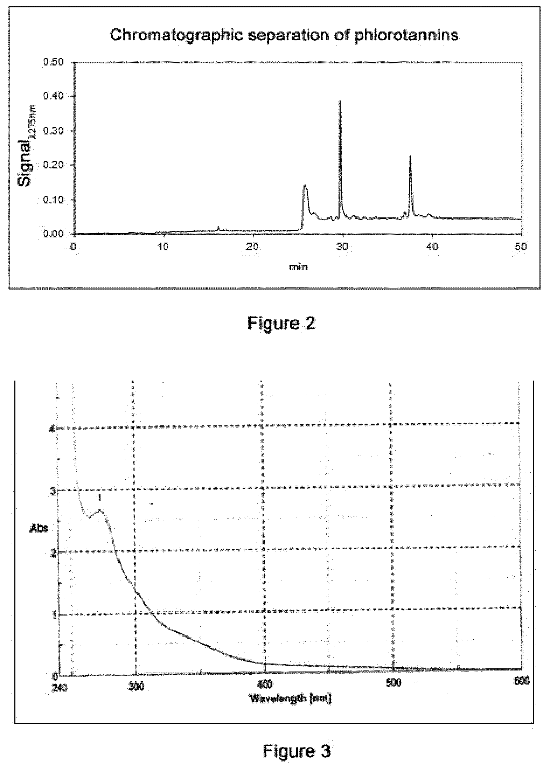

Maximum absorbance of this extract which is lyophilized and subsequently redissolved in water, with respect to a water blank is between 250 nm and 280 nm, as shown in Figure 3.

### Total polyphenol content:

The term polyphenols is understood to mean the entire family of compounds present in plants, which contain or are derived from the phenol group: benzoic acid derivatives, cinnamic acid derivatives, flavonoids, etc.

A slight modification of the method proposed in Colorimetry of total phenolics with phosphomolybdic phosphotungstic acid reagents. Singleton & Rossi, Singleton, V. L.; Rossi, J. J. Am. J. Enol. Vitic., 16, 144-158, 1965, was used to determine the polyphenol content of the obtained extract. Thus, 500 µl of sample dissolved in water was used, to which 2.5 ml of Folin-Ciocalteu reagent and 2.0 ml of Na₂CO₃ were added. Absorbance was read at 765 nm, after incubating the samples for 15 min at 45°C.

**Table 2.- Yields of aqueous extracts in dry and wet basis. d.b.: considering 24 h × 45°C drying**

| Species | Yield (%, w/w)_{d.b.} | Yield (%, w/w)_{w.b.} |
|---|---|---|
| *Ascophyllum nodosum* | 38.0 | 6.6 |
| *Bifurcaria bifurcata* | 27.7 | 4.8 |
| *Saccorhiza polyschides* | 42.0 | 4.9 |
| *Sargassum muticum* | 57.3 | 2.9 |

The inhibition assay of the DPPH radical proposed in "Use of a free radical method to evaluate antioxidant activity", Brand-Williams W, Cuvelier ME, Berset C. LWT Food Sci Technol 28:5-30 18 (1995), was used to determine the proton-donating capability of the extract. The method was applied with a slight modification as described, in which 20 µl of an aqueous solution of the extract were added to 980 µl of a methanolic solution of the radical (6.9 ×10⁻⁵ mol/l). The drop in absorbance was recorded at 515 nm after 16 minutes. The percentage of inhibition of the DPPH radical was calculated against the recording of a blank. Concentration ranges of between 5 and 40 mg/ml of *Ascophyllum nodosum* and *Bifurcaria bifurcata* extracts were evaluated to determine EC50, the results of which were 23.73 ± 3.83 mg extract/ml and 17.68± 2.2 mg extract/ml, respectively. These same values expressed as gallic acid equivalents (GAE) correspond to 1.08± 0.16 and 1.04 ± 0.13 mg GAE/ml, respectively.

**Table 3. Characterization of lyophilized extracts from four brown algae varieties.**

| Species | Total sugars | Sulfates | Uronic acid | Total polyphenols | Trace elements | | | | | |
|---|---|---|---|---|---|---|---|---|---|---|
| | | | | | Na | K | Mg | P | Ca | S |
| | mg/g extract | | | | | | | | | |
| *Ascophyllum nodosum* | 115 | 50 | 47 | 44 | 70 | 46 | 9 | 2 | 6 | 18 |
| *Bifurcaria bifurcata* | 156 | 118 | 55 | 62.4 | 20 | 71 | 3 | 1 | 4 | 36 |
| *Saccorhiza polyschides* | 33 | 44 | 10 | 3 | 48 | 180 | 6 | 4 | 3 | 12 |
| *Sargassum muticum* | 122 | 70 | 40 | 12 | 44 | 87 | 23 | 4 | 5 | 19 |

### Polysaccharide content

The term polysaccharides is understood to mean the polymers formed from sugars, primarily hexoses and/or pentoses, which function as a structural component and energy store in plants.

The polysaccharide content was determined by means of the phenol-sulfuric acid method proposed in Colorimetric method for determination of sugars and related substances, Dubois, M.; Gilles, K. A.; Hamilton, J. K.; Rebers, P. A.; Smith, F. Anal. Chem., 28, 350-356, 1956. An average carbohydrate content of 156.7 mg of glucose/g of lyophilizate of the extract obtained from the macroalga *Bifurcaria bifurcata* was obtained, being 29% greater than that obtained from *Ascophyllum nodosum* in similar conditions.

Fucoidans is understood to mean a family of polysaccharides characteristic of algae, which are formed by polymerization of derivatives of fucose, a sulfated sugar.

The average fucoidan content was determined by means of the method described in Determination of inorganic sulphate in studies on the enzymic and non-enzymic hydrolysis of carbohydrate and other sulphate esters, Dodgson, Dodgson, K. S., Biochem. J., 78, pp. 312-317, 1961, and is expressed as total sulfates after digesting the sample. The average value in the *Bifurcaria bifurcata* extracts is 118 mg of sulfates/g of lyophilisate, 134% greater than an *Ascophyllum nodosum* extract obtained in similar conditions. The sulfur levels determined by means of ICP-OES techniques (mean of 36.3 mg S/g of lyophilisate) were twice those obtained for *Ascophyllum nodosum* (mean of 18.14 mg/g).

The main polysaccharides in algae are the so-called alginates, formed by polymerization primarily of glucuronic acid.

The alginate content was estimated from the uronic acids content according to the method proposed in New method for quantitative determination of uronic acids, Blumenkrantz *et al.,* Blumenkrantz, N. and Asboe-Hansen, G. Anal. Biochem, 54, pp. 484-489, 1973. The *Bifurcaria bifurcata* extract showed a mean alginate content, expressed as uronic acids and using glucuronic acid as a standard, of 55.1 mg/g of lyophilizate, being 15% greater than an *Ascophyllum nodosum* extract obtained in the same conditions.

### Spectrophotometric determination of total polyphenols

The Folin-Ciocalteu method for determination of total polyphenols was used, using phloroglucinol as a standard. The average total polyphenol content, which polyphenols were primarily phlorotannins, in the *Bifurcaria bifurcata* extract, was of 62.4 mg equivalents of phloroglucinol/g of lyophilizate, 7.3% greater than that obtained from *Ascophyllum nodosum.*

### Chromatographic detection of phlorotannins:

The term phlorotannins is understood to mean polymers generated by polymerization of phloroglucinol (1,3,5-trihydroxybenzene), a parallelism with the name tannins, formed by polymerization of catechin/epicatechin and derivatives. Tannins are cell wall components in plants and phlorotannins are cell wall components in macroalgae.

Phlorotannins were extracted from the lyophilized extract, according to the methodology proposed in High-performance liquid chromatographic analysis of phlorotannins from the brown alga Fucus vesiculosus Phytochem, Koivikko *et al.*, 2007, Koivikko, R., Loponen, J., Pihlaja, K. and Jormalainen, V., Anal. 18, pp. 326-332, 2007, in which the steps of washing with hexane were eliminated.

95 mg of the *Bifurcaria bifurcata* extract were weighed and solubilized in 25 ml of water; the phlorotannins were subsequently extracted 4 times with 10 ml of an acetone/water: 7/3 mixture. The sample was centrifuged to separate the supernatant. The supernatant portions were pooled and acetone was removed with N₂ stream. The residue was resuspended in water and subsequently lyophilized. The lyophilisate was dissolved in 1 ml of distilled water and was ready to be injected into high-performance liquid chromatography equipment. The chromatographic separation method was the one proposed in Toxicity and antioxidant activity in vitro and in vivo of two Fucus vesiculosus extracts, Zaragoza, M.C., Lopez, D., Sáiz, M. P., Poquet, M., Pérez, J., Puig-Parellada, P., Mármol, F., Simonetti, P., Gardana, C., Lerat, Y., Burtin, P., Inisan, C., Rousseau, I., Besnard, M., and Mitjavila, M.T. J. Agric. Food Chem, 56, pp. 7773-7780, 2008.

Figure 2 shows the major peaks (5-6, 11 and 18) the signal of which is detected at 25-26, 30 and 37 min, respectively. Maximum absorbance of the phlorotannin mixture is observed at 273 nm, as shown in Figure 3. The UV-Visible spectrum profile coincides with the profile of a phloroglucinol molecule standard.

## Claims

1. A lyophilized antioxidant extract obtained from brown macroalgae **characterized by**:
a) a carbohydrate content between 33 and 156 mg of glucose/g of lyophilizate;
b) a fucoidan content, said fucoidans expressed as total sulfates after digesting the sample, between 44 and 118 mg of sulfates/g of lyophilizate;
c) a phlorotannin content, said phlorotannins expressed as phloroglucinol, between 12 and 62.4 mg equivalents of phloroglucinol/g of lyophilizate; and
d) an alginate content between 10 and 55 mg equivalents of glucuronic acid/g of lyophilizate.

2. The extract according to claim 1, **characterized by** a maximum absorbance in the ultraviolet region between 260-280 nm.

3. The extract according to claim 1, **characterized by** an inhibitory capacity of DPPH radical with an EC₅₀ value of 17.7 to 23.7 mg of extract/ml.

4. The extract according to claims 1 to 3, where the brown macroalga is *Bifurcaria bifurcata, Ascophyllum nodosum, Saccorhiza polyschides* and *Sargassum muticum.*

5. A method for obtaining a lyophilized antioxidant extract, characterized according to claims 1 to 4, from fresh or dry brown macroalgae, which comprises:
a) washing the macroalgae with water if fresh macroalgae are used;
b) mixing the macroalga with water with a liquid/solid (L/S) ratio between 3 and 5 g/g;
c) grinding the mixture obtaining a ground macroalga with a particle size less than 3 mm;
d) mixing the ground macroalga with a liquid solvent comprising pure ethanol or ethanol:water mixtures at a 1:1 v/v ratio, at a liquid/solid (L/S) ratio of between 5 and 15 (g/g) when starting from fresh macroalga and between 50 and 150 (g/g) when starting from dry macroalga;
e) subjecting the mixture to a cell disruption process by continuously applying ultrasound with a power density in the range between 3 and 13 W/cm³;
f) separating the solid algal residue obtained in the preceding step by means of sedimentation and subsequent centrifugation or filtration;
g) removing the ethanol or 50% of the water under vacuum at a temperature of less than 40°C, obtaining a concentrated extract; and
h) lyophilizing the concentrated extract obtaining a lyophilized extract.

6. The method according to claim 5, wherein the ground particle size is between 0.5 and 2 mm for dry macroalga and between 0.5 and 3 mm for fresh macroalga.

7. Use of the antioxidant extract characterized according to claims 1 to 4 as an ingredient in cosmetic and food formulations.

## Amended claims

### Amended claims under Art. 19.1 PCT

**1.** An aqueous antioxidant extract and its lyophilized derivative obtained from brown macroalgae **characterized by**:
a. a carbohydrate content between 33 and 156 mg of glucose/g of extract once lyophilized;
b. a fucoidan content, said fucoidans expressed as total sulfates after digesting the sample, between 44 and 118 mg of sulfates/g once lyophilized lyophilized;
c. a phlorotannin content, said phlorotannins expressed as phloroglucinol, between 12 and 62.4 mg equivalents of phloroglucinol/g once lyophilized lyophilized; and
d. an alginate content between 10 and 55 mg equivalents of glucuronic acid/g once lyophilized lyophilized.

**2.** The extract according to claim 1, **characterized by** a maximum absorbance in the ultraviolet region between 260-280 nm.

**3.** The extract according to claim 1, **characterized by** an inhibitory capacity of DPPH radical with an EC₅₀ value of 17.7 to 23.7 mg of extract/ml.

**4.** The extract according to claims 1 to 3, where the brown macroalga is *Bifurcaria bifurcata, Ascophyllum nodosum, Saccorhiza polyschides* and *Sargassum muticum.*

**5.** A method for obtaining an antioxidant extract, characterized according to claims 1 to 4, from fresh or dry brown macroalgae, which comprises:
a. washing the macroalgae with water if fresh macroalgae are used;
b. mixing the macroalga with water with a liquid/solid (L/S) ratio between 3 and 5 g/g;
c. grinding the mixture obtaining a ground macroalga with a particle size less than 3 mm;
d. mixing the ground macroalga with a liquid solvent comprising pure ethanol or ethanol:water mixtures at a 1:1 v/v ratio, at a liquid/solid (L/S) ratio of between 5 and 15 (g/g) when starting from fresh macroalga and between 50 and 150 (g/g) when starting from dry macroalga;
e. subjecting the mixture to a cell disruption process by continuously applying ultrasound with a power density in the range between 3 and 13 W/cm³;
f. separating the solid algal residue obtained in the preceding step by means of sedimentation and subsequent centrifugation or filtration;
g. removing the ethanol or 50% of the water under vacuum at a temperature of less than 40°C, obtaining a concentrated extract; and
h. lyophilizing the concentrated extract obtaining a lyophilized extract.

**6.** The method according to claim 5, wherein the ground particle size is between 0.5 and 2 mm for dry macroalga and between 0.5 and 3 mm for fresh macroalga.

**7.** Use of the antioxidant extract characterized according to claims 1 to 4 as an ingredient in cosmetic and food formulations. According to Art. 19.1 PCT, referred to the modification of the claims under the International Office and taken into account the citations and explanations contained in the Written Opinion issued by the International Search Authority, the applicant has made the following amendments on the set of claims:
a) Claim 1 has been amended in order to include the term "aqueous" which makes reference to the state in which the extract can be found and that is obtained as previous step to the obtention of the lyophilized extract.
b) Scientific names of the species named on claim 4 have been included in italic in said claim in order to comply with Rule 10.3 PCT.
c) Claim 5 has been amended by deleting the term "lyophilized".
The applicant declares that the amendments are supported by the description of the invention and that are clearly and unambiguously derived from the description of the invention.
